# EUROPEAN PATENT APPLICATION

(11) **EP 4 253 361 A1**
(43) Date of publication of application: **04.10.2023**
(21) Application number: 21898404.5
(22) Date of filing: 04.11.2021
(51) Int. Cl.: C07C 67/343, C07C 69/708, B01J 20/02

(54) **METHOD FOR PREPARING HETEROGENEOUS LINEAR CARBONATES BY MEANS OF BASIC ION EXCHANGE RESIN**

(30) Priority: 27.11.2020 KR 20200163293
(71) Applicant: Lotte Chemical Corporation, Seoul, 05551 (KR)
(72) Inventor: KIM, Jin Hyung, Daejeon 34110 (KR); BAEK, Mi Hwa, Daejeon 34110 (KR); KIM, Wang Gyu, Daejeon 34110 (KR); CHOI, Jong Myung, Daejeon 34110 (KR); HAN, Eun Hye, Daejeon 34110 (KR)
(74) Representative: Germain Maureau
(86) International application number: PCT/KR2021/015847
(87) International publication number: WO 2022/114578

(57) **Abstract**

The present specification provides a method of preparing a heterogeneous linear carbonate, including transesterifying an aliphatic alcohol and a symmetric linear carbonate in the presence of a catalyst, wherein the catalyst is a porous type basic ion exchange resin having an exchange capacity of 1 (eq/l-wet resin) or more and 1.5 (eq/l-wet resin) or less. The preparation method of the present specification provides a method of preparing a heterogeneous, symmetric linear carbonate and asymmetric linear carbonate in high yield without a process of reactive distillation.

## Description

### [Technical field]

The present specification relates to a preparation method that advantageously simultaneously obtains high yields of a heterogeneous linear carbonate, i.e. a symmetric linear carbonate and an asymmetric linear carbonate.

### [Background Art]

Linear carbonates such as ethyl methyl carbonate (EMC) and diethyl carbonate (DEC) are mainly used as solvents (electrolytes) for lithium secondary batteries, and the carbonates are mainly used as a solvent for lithium secondary batteries because the carbonates are superior in energy storage density, charge capacity, number of charge/discharge cycles, stability, and the like compared to conventional solvents.

As a method of preparing ethyl methyl carbonate and diethyl carbonate, a transesterification reaction of a mixture of a cyclic carbonate (ethylene carbonate or propylene carbonate) and ethanol and methanol is generally well known. For example, when any dialkyl carbonate among diethyl carbonate, ethyl methyl carbonate and dimethyl carbonate is prepared by transesterifying ethylene carbonate or propylene carbonate and ethanol or a mixture of ethanol and methanol, methyl glycol ether or ethyl glycol ether is generated as a by-product.

These reaction by-products tend to be in an azeotropic state with a desired product and are very difficult to separate by distillation, so that it is common to purify the desired product by introducing a new extraction separation method.

Meanwhile, an industrial preparation method of ethyl methyl carbonate and diethyl carbonate by a transesterification reaction using dimethyl carbonate (DMC) as a raw material for preparation is also known. As an example, a reactive distillation method of dimethyl carbonate and ethanol using a basic catalyst such as sodium methoxide (SME) as a transesterification catalyst has a problem in that equipment and energy costs are high. In addition, sodium methoxide, which is a water-repellent material and is highly dangerous and hazardous, and does not dissolve in products, causing problems such as column plugging and fouling in equipment.

As another example, as the reactive distillation is a method of utilizing extractive distillation using a gel type strong basic exchange resin catalyst as a transesterification catalyst, since extractive distillation requires the addition of a separation process for separating an extractant into a product, there is a problem in that equipment costs and energy costs are high. In addition, there are problems that deactivation proceeds rapidly due to loss of exchange groups and formation of fine powder caused by low catalyst strength, and internal pressure also rises, requiring a process of periodically introducing new catalysts, so that productivity decreases and production costs rise.

Most of the methods of preparing ethyl methyl carbonate and diethyl carbonate using a cyclic carbonate or dimethyl carbonate (DMC) as raw materials are transesterification reactions using chemical equilibrium reactions. Then, in order to advance this reaction efficiently, a method of distilling off alkylene glycol and methanol, which are reaction by-products, is performed. However, at atmospheric pressure, for example, since it is practically impossible for DMC to form an azeotropic mixture with methanol, which has an approximate composition of 70% methanol and 30% DMC, and it is practically impossible to completely react DMC when performing this kind of transesterification reaction, in the actual reaction, DMC was also distilled off along with methanol, and as a result, the DMC conversion rate was insufficient or industrial reproducibility was low. Therefore, a separate method for separating and removing methanol from the azeotropic mixture of DMC and methanol is required.

Therefore, a new preparation method with a high DMC conversion rate is required.

### [Related Art Document]

### [Patent Document]

(Patent Document 1) Japanese Unexamined Patent Publication No. 2010-168365

### [Disclosure]

### [Technical Problem]

The present invention relates to a preparation method for advantageously obtaining heterogeneous linear carbonates, i.e., a symmetric linear carbonate and an asymmetric linear carbonate simultaneously, with high selectivity, by performing a transesterification reaction between an aliphatic alcohol and a symmetric linear carbonate at a high conversion rate without separate reactive distillation and distilling the symmetric linear carbonate out of the reaction system as an azeotropic mixture of reaction by-products.

### [Technical Solution]

One embodiment of the present invention provides a method of preparing a heterogeneous linear carbonate, including transesterifying an aliphatic alcohol and a symmetric linear carbonate in the presence of a catalyst, wherein the catalyst is a porous type basic ion exchange resin having an exchange capacity of 1 (eq/l-wet resin) or more and 1.5 (eq/l-wet resin) or less.

### [Advantageous Effects]

A preparation method according to one embodiment of the present invention can simultaneously prepare a heterogeneous, symmetric linear carbonate and asymmetric linear carbonate in high yield without a process of reactive distillation.

In addition, in the preparation method according to one embodiment of the present invention, since the surface area is relatively widened by using a porous type basic ion exchange resin as a catalyst, and thus the contact with the reactant can be maximized, heterogeneous symmetric linear carbonates and asymmetric linear carbonates can be prepared with high reaction rates and high reaction efficiencies.

In addition, since the catalyst used in one embodiment of the present invention has excellent decoloring properties, it is possible to relatively reduce the washing time of the ion exchange resin and the amount of washing solvent used at the beginning of the reaction, and due to its excellent strength, the loss of exchange groups and formation of fine powders are reduced during reuse, so that the reuse cycle is long and productivity can be improved.

### [Modes of the Invention]

Advantages and features of the present invention and methods of achieving them will become apparent with reference to the embodiments described below in detail. However, the present invention is not limited to the embodiments disclosed below and may be embodied in various different forms, and the embodiments merely serve to complete the disclosure of the present invention and to fully inform the scope of the invention to those skilled in the art to which the invention pertains, which is defined only by the spirit of the claims. Throughout the specification, like reference numerals refer to like components.

Unless otherwise defined, all terms (including technical and scientific terms) used in the present specification may be used in a meaning commonly understood by those of ordinary skill in the art to which the present invention belongs. In addition, terms defined in commonly used dictionaries are not interpreted ideally or excessively unless explicitly specifically defined.

Throughout the specification, when a part is said to "include" a component, this means that the part may further include other components rather than excluding other components unless specifically stated to the contrary.

Hereinafter, the present invention will be described in detail.

One embodiment of the present invention provides a method of preparing a heterogeneous linear carbonate, including transesterifying an aliphatic alcohol and a symmetric linear carbonate in the presence of a catalyst, wherein the catalyst is a porous type basic ion exchange resin having an exchange capacity of 1 (eq/l-wet resin) or more and 1.5 (eq/l-wet resin) or less.

In one embodiment of the present invention, the porous type basic ion exchange resin is a porous strong basic ion exchange resin. The porous strong basic ion exchange resin is an ion type, and may include a bromine anion (Br⁻), a thiocyanate anion (SCN⁻), a chlorine anion (Cl⁻), an acetate anion (CH₃COO⁻), a hydroxy group anion (OH⁻), a fluorine anion (F⁻), and the like. The porous weak basic ion exchange resin is an ion type, and may include a citrate anion (C₃H₅O(COO)₃³), sulfate (SO₄²⁻), and the like.

In the present specification, the aliphatic alcohol is not particularly limited, but may include alcohols having 1 to 10 carbon atoms, specifically methanol, ethanol, propanol, isopropanol, butanol, isobutanol, and the like. In one embodiment of the present specification, the aliphatic alcohol is ethanol.

In one embodiment of the present invention, the symmetric linear carbonate, which is a reactant in the transesterification reaction, is dimethyl carbonate (DMC).

In one embodiment of the present invention, heterogeneous linear carbonates produced after the transesterification reaction are symmetric linear carbonates and asymmetric linear carbonates.

In one embodiment, the asymmetric linear carbonate produced after the transesterification reaction is ethyl methyl carbonate (EMC).

In one embodiment, the symmetric linear carbonate produced after the transesterification reaction is diethyl carbonate (DEC).

According to one embodiment of the present invention, the transesterification reaction produces a heterogeneous linear carbonate, namely an asymmetric linear carbonate and an asymmetric linear carbonate. The asymmetric linear carbonate of the product in the transesterification reaction is ethyl methyl carbonate (EMC), and the symmetric linear carbonate is diethyl carbonate (DEC).

In the preparation method according to one embodiment of the present invention, a porous type basic ion exchange resin is used as a catalyst in a transesterification reaction between an aliphatic alcohol and a symmetric linear carbonate. Since the porous type basic ion exchange resin has many (about 10 to 1000) macro pores on its surface, the resin is chemically stable compared to the gel-type strong basic ion exchange resin and can maximize contact with a reactant due to a large surface area, so that the reaction rate is fast and the internal diffusion rate is excellent, and it is possible to simultaneously prepare heterogeneous linear carbonates with high reaction efficiency.

In addition, a heat resistance temperature of the porous type basic ion exchange resin is about 40 °C to 100 °C, and an operating range is wider than the heat resistance temperature of about 40 °C to 80 °C of the gel type strong basic ion exchange resin, so that there is little risk that the pH of the product will increase due to the elution of the exchange group in the event of a runaway reaction such as an exothermic reaction.

In addition, since the porous type basic ion exchange resin used in one embodiment of the present invention has excellent decoloring properties, it is possible to relatively reduce the washing time of the ion exchange resin and the amount of washing solvent used at the beginning of the reaction, and due to its excellent strength, the loss of exchange groups and formation of fine powders are reduced during reuse, so that the reuse cycle is long and productivity can be improved.

In one embodiment of the present invention, the catalyst is a porous type basic ion exchange resin having an exchange capacity of 1 (eq/l-wet resin) or more and 1.5 (eq/l-wet resin) or less. In general, the higher the degree of crosslinking of the ion exchange resin, the more exchange groups are attached to the matrix, so that the exchange capacity tends to be high, but the reaction efficiency decreases because the exchange groups participating in the reaction decrease due to a strong bond between the matrix and the exchange groups. When the exchange capacity of the catalyst exceeds 1.5 (eq/l-wet resin), the reaction rate is too slow, and when the exchange capacity of the catalyst is less than 1 (eq/l-wet resin), the reaction rate is fast, but the bond between the matrix and the exchange groups is weakened, resulting in a decrease in strength against rapid volume change.

In the present specification, the exchange capacity means that the ability of an ion exchange resin to adsorb ions is expressed as an equivalent amount of ions obtained by adsorbing a certain volume of the resin, that is, may mean an exchange amount that can be removed by 1 liter of resin.

In the present specification, the unit of the exchange capacity is eq/l-wet resin, and means a value expressed as an equivalent of exchange capacity per liter of the ion exchange resin.

In one embodiment of the present invention, an exchange group of the basic ion exchange resin is trimethylammonium (TMA) or dimethyl ethanol ammonium (DMEA), and an ion type is a chloride anion (Cl⁻) or a hydroxy group anion (OH⁻).

In one embodiment of the present specification, a matrix of the basic ion exchange resin is a copolymer of styrene and divinylbenzene.

In one embodiment, the transesterification reaction is performed at a temperature of 40 °C or more and 100 °C or less. More specifically, the transesterification reaction is performed at a temperature of 40 °C or more and 80 °C or less. When the transesterification reaction is performed below 40 °C, there is a problem in that the reaction rate is lowered, and when the transesterification reaction is performed above 100 °C, there are problems that an exchange group of the strong basic ion exchange resin is decomposed and the activity is lowered, and the reuse cycle is shortened and the pH of the product increases.

In one embodiment of the present invention, in the step of transesterifying an aliphatic alcohol and a symmetric linear carbonate in the presence of a catalyst, a mass ratio of the aliphatic alcohol and the symmetric linear carbonate (aliphatic alcohol: symmetric linear carbonate) is 1:10 or more and 10:1 or less. Specifically, in another embodiment, the mass ratio of the aliphatic alcohol and the symmetric linear carbonate (aliphatic alcohol: symmetric linear carbonate) is 1:2 or more and 5:1 or less. In the transesterification reaction, when a mass ratio of the aliphatic alcohol and the symmetric linear carbonate is less than 1:10, since the amount of aliphatic alcohol is small, the symmetric linear carbonate remains in excess, and the catalyst is not sufficiently swelled, the contact with the catalyst is lowered and the efficiency is lowered, resulting in a decrease in productivity, and when the mass ratio of the aliphatic alcohol and the symmetric linear carbonate exceeds 10:1, after the reaction, a size of the distillation process to separate the heterogeneous symmetric linear carbonate (DEC) and asymmetric linear carbonate (EMC) and aliphatic alcohol increases, and as the aliphatic alcohol and asymmetric linear carbonate (EMC) are azeotropes, the larger the amount of aliphatic alcohol, the more columns with high separation efficiency are required, a lot of energy is consumed in the decompression and/or pressurization process, a large amount of asymmetric linear carbonate (EMC) is lost to the top of the distillation process, which makes the process unproductive.

In one embodiment of the present invention, the transesterification reaction is performed in a fixed bed reactor or a continuous stirred tank reactor (CSTR).

In one embodiment of the present invention, the transesterification reaction is performed in the fixed bed reactor. In this case, operation is simple and energy costs can be saved.

In one embodiment of the present invention, the fixed bed reactor may be operated in a series type. In one embodiment, the fixed bed reactor may be a reactor composed of one or more and four or less reactors in series.

Meanwhile, the method of preparing a heterogeneous linear carbonate according to the present invention may further include conventional steps known in the art before or after each of the above steps, in addition to the above steps.

Hereinafter, examples will be given to describe the present invention in detail. However, the examples according to the present invention may be modified in various other forms, and the scope of the present invention is not to be construed as being limited to the examples described below. The examples of the present specification are provided to more completely explain the present invention to those of ordinary skill in the art.

### Example 1

A fixed bed reactor was used as a series 4, and 80 cc (58 g) of a porous type basic anion exchange resin having trimethylammonium (TMA) as an exchange group and a chlorine anion (Cl-) as an ion type was charged in each reactor. An exchange capacity (equivalent/l-wet resin per unit volume) was 1.3, and Samyang Corporation's TRILITE AMP-18 product was used. A mass ratio of ethanol to DMC was 0.8 and the reactor was operated continuously at 50 °C at 2 cc/min.

### Example 2

The reactor was operated in the same manner as in Example 1, except that a reaction temperature was set to 80 °C instead of 50 °C in Example 1.

### Example 3

The reactor was operated in the same manner as in Example 1, except that Samyang Corporation's TRILITE AMP-14, whose catalyst exchange capacity is 1.0 (eq/l-wet resin) instead of 1.3 (eq/l-wet resin), was used, and the reaction temperature was 80 °C instead of 50 °C.

### Example 4

The reactor was operated in the same manner as in Example 1, except that Samyang Corporation's TRILITE AMP-14, whose catalyst exchange capacity is 1.0 (eq/l-wet resin) instead of 1.3 (eq/l-wet resin), was used, a mass ratio of ethanol to DMC was 0.3 instead of 0.8, and the reaction temperature was 70 °C instead of 50 °C.

### Example 5

The reactor was operated in the same manner as in Example 1, except that Samyang Corporation's TRILITE AMP-14, whose catalyst exchange capacity is 1.0 (eq/l-wet resin) instead of 1.3 (eq/l-wet resin), was used, a mass ratio of ethanol to DMC was 2 instead of 0.8, and the reaction temperature was 70 °C instead of 50 °C.

### Example 6

The reactor was operated in the same manner as in Example 1, except that Samyang Corporation's TRILITE AMP-14, whose catalyst exchange capacity is 1.0 (eq/l-wet resin) instead of 1.3 (eq/l-wet resin), was used, a mass ratio of ethanol to DMC was 4 instead of 0.8, and the reaction temperature was 70 °C instead of 50 °C.

### Example 7

The reactor was operated in the same manner as in Example 4, except that a mass ratio of ethanol and DMC was 0.05 instead of 0.3 in Example 4.

### Example 8

The reactor was operated in the same manner as in Example 1, except that a reaction temperature was set to 120 °C instead of 50 °C in Example 1.

### Comparative Example 1

The reactor was operated in the same manner as in Example 1, except that, in Example 1, Samyang Corporation's TRILITE SAR-10 product (exchange capacity is 1.3 (eq/l-wet resin)), which is a gel type (micropore) strong basic ion exchange resin having trimethylammonium (TMA) as an exchange group instead of a porous type basic anion exchange resin catalyst, and a chloride anion (Cl⁻) as an ion type, was used.

### Comparative Example 2

The reactor was operated in the same manner as in Comparative Example 1, except that a reaction temperature was set to 120 °C instead of 50 °C in Comparative Example 1.

### Comparative Example 3

The reactor was operated in the same manner as in Example 1, except that, in Example 1, Samyang Corporation's DIAION WA-20 product (exchange capacity is 2.5 (eq/l-wet resin)), which is a porous type basic anion exchange resin having a secondary amine as an exchange group and a hydroxyl anion (OH⁻) as an ionic type, instead of a porous type basic anion exchange resin catalyst having trimethylammonium (TMA) as an exchange group and a chlorine anion (Cl-) as an ion type, was used.

The results of the reactor operation of Examples 1 to 8 and Comparative Examples 1 to 3 are shown in Table 1 below.

**[Table 1]**

| Classification | | Ex. 1 | Ex. 2 | Ex. 3 | Ex. 4 | Ex. 5 | Ex. 6 | Ex. 7 | Ex. 8 | Com p. Ex. 1 | Com p. Ex. 2 | Comp . Ex. 3 |
|---|---|---|---|---|---|---|---|---|---|---|---|---|
| Conditi ons | Exchange group | TMA | TMA | TMA | TMA | TMA | TMA | TMA | TMA | TMA | TMA | SEA |
| | Ion type | Cl⁻ | Cl⁻ | Cl⁻ | Cl⁻ | Cl⁻ | Cl⁻ | Cl⁻ | Cl⁻ | Cl⁻ | Cl⁻ | OH⁻ |
| | Type | Porou s | Porou s | Porou s | Porou s | Porou s | Porou s | Porou s | Porou s | Gel type | Gel type | Porou s |
| | Exchange capacity 1 (eq/1-wet resin) | 1.3 | 1.3 | 1.0 | 1.0 | 1.0 | 1.0 | 1.3 | 1.3 | 1.3 | 1.3 | 2.5 |
| | EtOH/DMC Mass ratio | 0.8 | 0.8 | 0.8 | 0.3 | 2 | 4 | 0.05 | 0.8 | 0.8 | 0.8 | 0.8 |
| | Reaction temperature (°C) | 50 | 80 | 80 | 70 | 70 | 70 | 70 | 120 | 50 | 80 | 80 |
| Results | DMC conversion | 64 | 68 | 71 | 66 | 87 | 94 | 20 | 58 | 50 | 45 | 10 |
| | rate (%) | | | | | | | | | | | |
| | EMC selectivity (%) | 75 | 70 | 67 | 72 | 53 | 47 | 92 | 80 | 82 | 87 | 98 |
| | DEC selectivity [%] | 25 | 30 | 33 | 28 | 47 | 53 | 8 | 20 | 18 | 13 | 2 |

| | | | | | | | | | | | | |
|---|---|---|---|---|---|---|---|---|---|---|---|---|
| *Ex.: Example; Comp. Ex.: Comparative Example | | | | | | | | | | | | |

In Table 1, TMA is trimethylammonium, SEA is secondary amine, DMC is dimethyl carbonate, and EMC is ethyl methyl carbonate.

Referring to the results of Table 1, it was confirmed that a preparation method according to one embodiment of the present invention had a high DMC conversion rate by using a porous type basic ion exchange resin as a catalyst. In particular, when comparing Example 1 and Comparative Example 1, and Example 2 and Comparative Example 2 in detail, it was confirmed that, under the same conditions, the case where the porous type basic ion exchange resin was used, has a higher DMC conversion rate than the case where the gel type basic ion exchange resin was used. More specifically, it was confirmed that, in the case of Example 2 using the porous type basic ion exchange resin at 80 °C, the DMC conversion rate was high, and in the case of Comparative Example 2 using the gel type basic ion exchange resin, elution of the exchange group progressed, resulting in low DMC conversion.

In addition, it was confirmed that, when comparing Example 2 and Example 8, even when the same porous type basic ion exchange resin was used, when the reaction was performed at 120 °C exceeding the heat resistance temperature range, elution of the exchange group progressed, resulting in low DMC conversion. Therefore, it is appropriate that the reaction temperature be performed at a temperature of 40 °C or more and 100 °C or less.

Referring to Examples 2 and 3 and Comparative Example 3, it was confirmed that under the same conditions, when an exchange capacity is low, the binding of the exchange group to the matrix is weak, and the exchange group participating in the reaction increases, so that the DMC conversion rate increases.

In addition, referring to Examples 4 to 7, it was confirmed the yield of heterogeneous symmetric linear carbonate (DEC) and asymmetric linear carbonate (EMC) as products can be controlled by controlling a mass ratio of aliphatic alcohol and symmetric linear carbonate (aliphatic alcohol:symmetric linear carbonate), that is, a mass ratio of ethanol and DMC (EtOH:DMC). However, in the case of Example 7, it was confirmed that a mass ratio of ethanol to symmetric linear carbonate (DMC) is less than 0.1, and an amount of reactive ethanol is small, leaving an excessive amount of DMC and not sufficiently swelling the catalyst, so that the contact with the catalyst is lowered and the DMC conversion rate is lowered. Therefore, it is preferred that a mass ratio of ethanol to a symmetric linear carbonate (DMC) is less than 0.1 and 10 or more.

## Claims

1. A method of preparing a heterogeneous linear carbonate, comprising transesterifying an aliphatic alcohol and a symmetric linear carbonate in the presence of a catalyst,
wherein the catalyst is a porous type basic ion exchange resin having an exchange capacity of 1 (eq/l-wet resin) or more and 1.5 (eq/l-wet resin) or less.

2. The method of claim 1, wherein an exchange group of the basic ion exchange resin is trimethylammonium (TMA) or dimethyl ethanol ammonium (DMEA), and
an ion type is a chlorine anion (Cl⁻) or a hydroxy group anion (OH⁻).

3. The method of claim 1, wherein in the transesterification reaction, a mass ratio of the aliphatic alcohol and the symmetric linear carbonate (aliphatic alcohol: symmetric linear carbonate) is 1:10 or more and 10:1 or less.

4. The method of claim 1, wherein the transesterification reaction is performed in a fixed bed reactor or a continuous stirred tank reactor (CSTR).
